# EUROPEAN PATENT SPECIFICATION

(11) **EP 2 826 501 B1**
(45) Date of publication and mention of the grant of the patent: **18.01.2017**
(21) Application number: 13761184.4
(22) Date of filing: 13.03.2013
(51) Int. Cl.: A61B 5/155, A61B 5/15, A61M 1/02, A61B 5/154

(54) **CONTAINER FOR TESTING BLOOD AND BLOOD SAMPLING INSTRUMENT**
BEHÄLTER ZUR UNTERSUCHUNG VON BLUT UND BLUTENTNAHMEVORRICHTUNG
RÉCIPIENT POUR ANALYSE DE SANG ET INSTRUMENT DE PRÉLÈVEMENT SANGUIN

(30) Priority: 14.03.2012 JP 2012057502
(43) Date of publication of application: 21.01.2015
(73) Proprietor: Terumo Kabushiki Kaisha, Tokyo 151-0072 (JP)
(72) Inventor: HAYAKAWA Aya, Fujinomiya-shi Shizuoka 418-0004 (JP); AKIYAMA Masahiro, Fujinomiya-shi Shizuoka 418-0004 (JP)
(74) Representative: TBK
(86) International application number: PCT/JP2013/057100
(87) International publication number: WO 2013/137360

(56) References cited:
- EP-A1- 2 394 678
- WO-A1-2011/093260
- JP-A- H11 197 236
- JP-A- 2009 100 868
- JP-B2- 4 839 198
- JP-B2- 4 879 704
- US-B1- 6 592 613

## Description

The present invention relates to a container for testing blood which draws (stores) an initial blood flow to be tested, according to the preamble of claim 1, the features of which are known from e.g. document JP 4 839 198 B2, and a blood drawing instrument that is provided with the container for testing blood.

Usually, as described in document JP 2003-505185 A, for example, the blood drawing instrument is provided with: a vein puncture needle; a blood donor bag that stores blood drawn through the vein puncture needle; a plastic pipe that connects the vein puncture needle with the blood donor bag; a pipe segment that branches from a midway of the plastic pipe; and a container (the container for testing blood) that communicates with the plastic pipe via the pipe segment.

In using the blood drawing instrument of document JP 2003-505185 A, before collecting (storing) blood from a donor (a blood donor) into the blood donor bag, that is, before actual blood collection, an initial blood flow of the donor is drawn (inflows) from an inflow port that is disposed at a bottom part of the container for testing blood into an interior chamber of the container for testing blood. Then, the thus drawn (stored) initial blood flow is collected (outflows) into a blood sampling vial that communicates with an outflow port disposed at a top part of the container for testing blood, which is used for various tests.

Thus, the blood drawing instrument of document JP 2003-505185 A has an advantage of being able to draw (store) the initial blood flow that is to be used for the various tests easily. Also, the blood drawing instrument of document JP 2003-505185 A has an advantage that, even if bacteria that are present on or under skin at the time of blood withdrawal are mixed into the blood,
the blood contaminated with bacteria can be removed as the initial blood flow into an initial blood flow bag, and thus,
the bacterial contamination of the blood that is collected (stored) in the blood donor bag can be prevented.

However, according to the blood drawing instrument of document JP 2003-505185 A, an operator (a person who draws the blood) starts drawing the initial blood flow into the container for testing blood, and visually confirms, by a fluid level of the initial blood flow, that a predetermined amount of the initial blood flow is collected into the container, and thereafter, the operator closes a clamp provided in the pipe segment so as to stop (complete) the inflow of the initial blood flow into the container for testing blood. Thus, according to the blood drawing instrument of document JP 2003-505185 A, since the inflow of the initial blood flow is stopped manually, a timing of closing the clamp is varied according to the operator, and thus, variation in amount of the initial blood flow collected (stored) into the container for testing blood is caused, that is, an overmuch amount of the initial blood flow may be collected or even a minimum collecting quantity may not be not secured. Then, if the overmuch amount of the initial blood flow is collected, health damage or a loss of residual blood may be brought to the donor. On the other hand, when the minimum collecting quantity is not secured, the various tests cannot be performed with the initial blood flow, and therefore, safety of the blood collected into the blood donor bag cannot be assured. In particular, unless the minimum collecting quantity is secured, a blood product cannot be manufactured with the blood collected by the blood drawing instrument, which leads to a loss of the blood product.

Moreover, according to the blood drawing instrument of document JP 2003-505185 A, in prior to the inflow of the initial blood flow into the container for testing blood, air in the plastic pipe and the pipe segment inflows into the container for testing blood. Since the blood drawing instrument of document JP 2003-505185 A is not provided with a means that discharges such air in the container, the air remains in the container after completing the inflow of the initial blood flow. Thus, the blood drawing instrument of document JP 2003-505185 A has a problem that, when the initial blood flow is allowed to outflow into the blood sampling vial (the testing instrument) while a whole of the container for testing blood is placed horizontally (in horizontally placed state) so that a surface of the container is in contact with a work table or the like, an outflow port is opened to an air-remaining region in the container, and the air is entrapped into the initial blood flow that is allowed to outflow from the outflow port. When the air entrainment is caused as described above, during the outflow of the initial blood flow, the amount of the initial blood flow collected by the blood drawing instrument is decreased, so that the various tests cannot be performed with the initial blood flow similarly to the above-described case.

Documents EP 2 394 678 A1, US 6 592 613 B1 and JP 4 839 198 B2 disclose generic liquid or blood component collecting devices.

In all these devices of the prior art, a blood inflow port, a blood outflow port and a blood drawing part with respect to the container are arranged on the same side.

The object of the present invention is to provide a container for testing blood which does not cause a variation in amount of the initial blood flow collected into the container, can secure the minimum collecting quantity, and does not entrap the air even when the initial blood flow is allowed to outflow to the testing instrument while the container for testing blood is in the horizontally placed state, and aims to provide a blood drawing instrument provided with the container for testing blood.

The object of the invention is achieved by a container for testing blood according to claim 1. Advantageous embodiments are carried out according to the dependent claims.

According to the structure of the claimed container the first discharge part that has the hydrophilic bacteria-impermeable first filter is provided, the first discharge part and the blood outflow port are disposed at the end part of the main container body on the same side, and the blood drawing part that is provided with the cylindrical holder is connected to the blood outflow port, whereby, while the container for testing blood is in the horizontally placed state, the blood outflow port is disposed at a higher position (upper) than the blood inflow port. As a result, air, which is gathered to an upper of the internal space according to the inflow of the initial blood flow, is discharged from the first discharge part through the hydrophilic bacteria-impermeable first filter, and the inflow of the initial blood flow is automatically stopped when the initial blood flow becomes in contact with the hydrophilic bacteria-impermeable first filter, whereby the inflow is completed. Then, the internal space is filled only with the initial blood flow, and residual air is not resulted. Also, since the initial blood flow is not in contact with the hydrophilic bacteria-impermeable first filter before completing the inflow of the initial blood flow, air block of the hydrophilic bacteria-impermeable first filter due to the contact of the initial blood flow is not caused, whereby stop of the inflow of the initial blood flow due to the air block can be prevented.

Moreover, according to the container for testing blood, the first discharge part further includes a defoaming filter on the internal space side of the bacteria-impermeable first filter.

According to the above-described structure, by including the defoaming filter, even when an air bubble is contained in the initial blood flow, such a bubble is allowed to burst, and thus, a blood film is not generated by the foaming of the initial blood flow, or the bacteria-impermeable first filter is not air-blocked by such a blood film. As a result, the stop of the inflow of the initial blood flow due to such air block can be prevented.

Further, the container for testing blood includes a second discharge part which is disposed at an end part of the main container body on an opposite side of the first discharge part, is disposed at an upper end side of the main container body when being used so that the blood outflow port is directed vertically, communicates with the internal space, has a hydrophilic bacteria-impermeable second filter, and discharges the air in the internal space.

According to the above-described structure, the second discharge part that has the hydrophilic bacteria-impermeable second filter is provided, and, when using the container for testing blood so that the blood outflow port is directed vertically, that is, when the container for testing blood is hung down from a stand or the like so as to be in a drooping state, the second discharge part is positioned at an upper end side of the main container body, whereby the air, which is gathered to the upper of the internal space according to the inflow of the initial blood flow, is discharged from the second discharge part, and then, the inflow of the initial blood flow is automatically stopped when the initial blood flow becomes in contact with the hydrophilic bacteria-impermeable second filter. Then, the internal space is filled only with the initial blood flow, and the residual air is not resulted.

Also, the container for testing blood is the container for testing blood according, in which the second discharge part further includes a defoaming filter on the internal space side of the bacteria-impermeable second filter.

According to the above-described structure, by including the defoaming filter, even when an air bubble is contained in the initial blood flow, such a bubble is allowed to burst, and thus, a blood film is not generated by the foaming of the initial blood flow, or the bacteria-impermeable second filter is not air-blocked by such a blood film. As a result, the stop of the inflow of the initial blood flow due to such air block can be prevented.

The blood drawing instrument according to the present invention includes a blood drawing needle; a storage part that stores blood drawn through the blood drawing needle; a blood drawing line that connects the blood drawing needle and the storage part; a branching line that branches from a midway of the blood drawing line; and the container for testing blood which communicates with the blood drawing line via the branching line.

According to the above-described structure, by including the container for testing blood, in a state where the internal space is filled with the first blood flow and the air residue is absent, the inflow of the initial blood flow is automatically stopped. Also, even when the container for testing blood is in the horizontally placed state, the initial blood flow is not in contact with the bacteria-impermeable first filter or the bacteria-impermeable second filter before completing the inflow (the drawing) of the initial blood flow, whereby the air block of the bacteria-impermeable first filter or the bacteria-impermeable second filter due to the contact of the initial blood flow is not caused, so that the stop of the inflow of the initial blood flow due to such air block can be prevented.

Moreover, according to blood drawing instrument, the storage part is a blood bag or a blood separator.

Since the storage part is the blood bag or the blood separator, the blood drawing instrument can be utilized as a blood bag system or an apheresis collection set.

According to the container for testing blood and the blood drawing instrument according to the present invention, a variation in amount of the initial blood flow that is collected into the container is not caused, the minimum collecting quantity can be secured, and even if the initial blood flow is allowed to outflow to the testing instrument while the container for testing blood is in the lateral state, the air entrainment is not caused.

### Brief Description of Drawings

Fig. 1 is a cross-sectional view that illustrates a structure of an embodiment of a container for testing blood.
Fig. 2(a) is a cross-sectional view that illustrates a state of an inflow of an initial blood flow when using the container for testing blood in a lateral state. Fig. 2(b) is a cross-sectional view taken along an X-X line in Fig. 2(a). Fig. 2(c) is a cross-sectional view taken along the X-X line in another embodiment.
Fig. 3 is a cross-sectional view that illustrates a structure of another embodiment of the container for testing blood.
Figs. 4 (a) to 4(c) are perspective views that illustrate a structure of an end part of a first discharge part or a second discharge part.
Fig. 5 is a schematic view that illustrates a structure of an embodiment of a blood drawing instrument.

An embodiment of the container for testing blood according to the present invention will be described in detail with reference to the accompanying drawings.

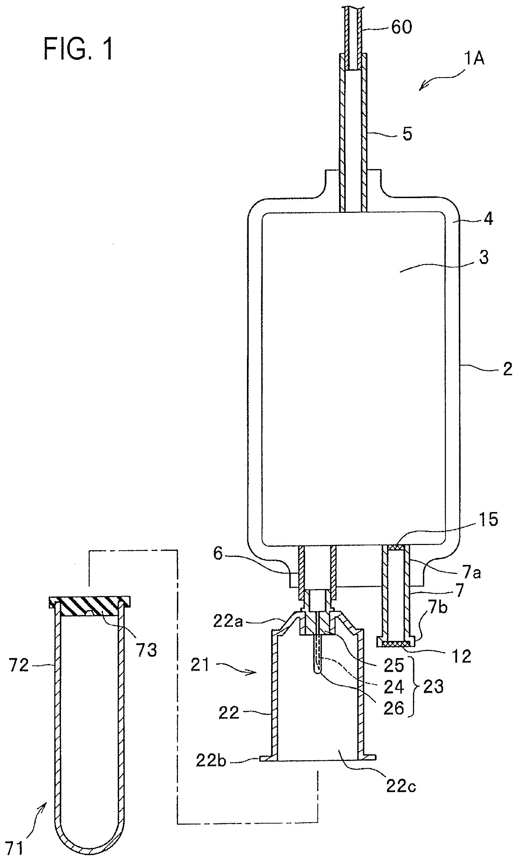

As illustrated in Fig. 1, a container for testing blood 1A includes a main container body 2, a blood inflow port 5, a blood outflow port 6, a blood drawing part 21 and a first discharge part 7.

The main container body 2 includes, inside itself, an internal space 3 that stores (collects) an initial blood flow that is to be used for a test, and is a bag-shaped container obtained by: superimposing seat materials that are preferably made of soft resin such as polyvinyl chloride; and forming a sealing part 4 at a periphery of the superimposed seat materials by fusion (heat seal, high frequency fusion or the like) or adhesion. Then, the internal space 3 is set to have a size that can store a preset amount of blood (for example, 25 ml).

At an end part of the main container body 2, the blood inflow port 5, the blood outflow port 6, the blood drawing part 21 that is connected to the blood outflow port 6, and the first discharge part 7 are disposed so as to communicate with the internal space 3. Then, when the container for testing blood 1A is hung down from a stand or the like so as to be in a drooping state, the blood inflow port 5 is disposed at an upper end side of the main container body 2, and the blood outflow port 6, the blood drawing part 21 and the first discharge part 7 are disposed at a bottom end side of the main container body 2.

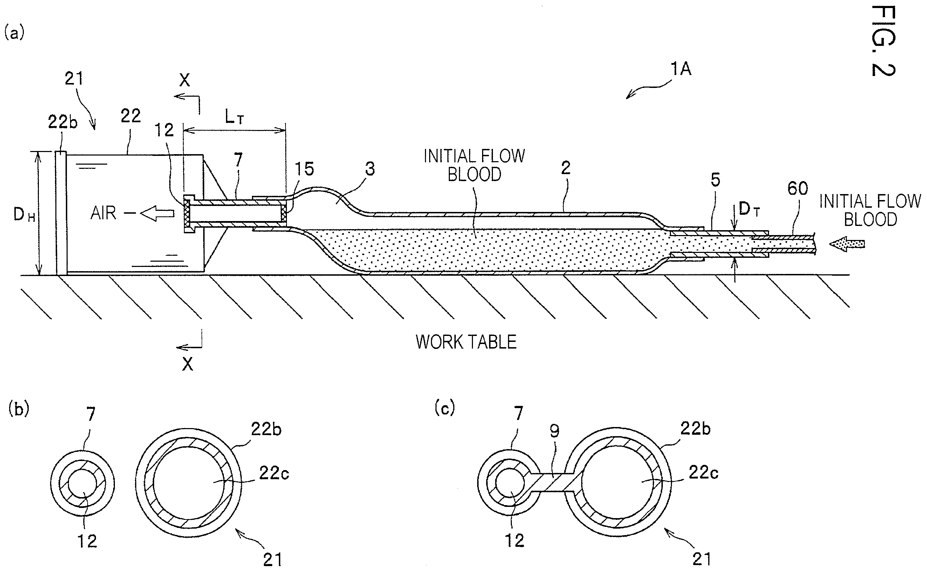

The respective dispositions of the blood inflow port 5, the blood outflow port 6, the blood drawing part 21 and the first discharge part 7 are not limited to the dispositions illustrated in Fig. 1, and, for example, while the main container body 2 (the container for testing blood 1A) is in the lateral state, as long as the first discharge part 7 and the blood drawing part 21 are disposed on the same side, and the first discharge part 7 is disposed at a higher position than that of the blood inflow port 5 (see Fig. 2(a)), the blood outflow port 6, the blood drawing part 21 and the first discharge part 7 may be disposed at the upper end side of the main container body 2 where the blood inflow port 5 is disposed (not illustrated). Incidentally, the "lateral state" means a state where a whole of the container for testing blood 1A is placed horizontally so that a surface of the container is in contact with a work table or the like.

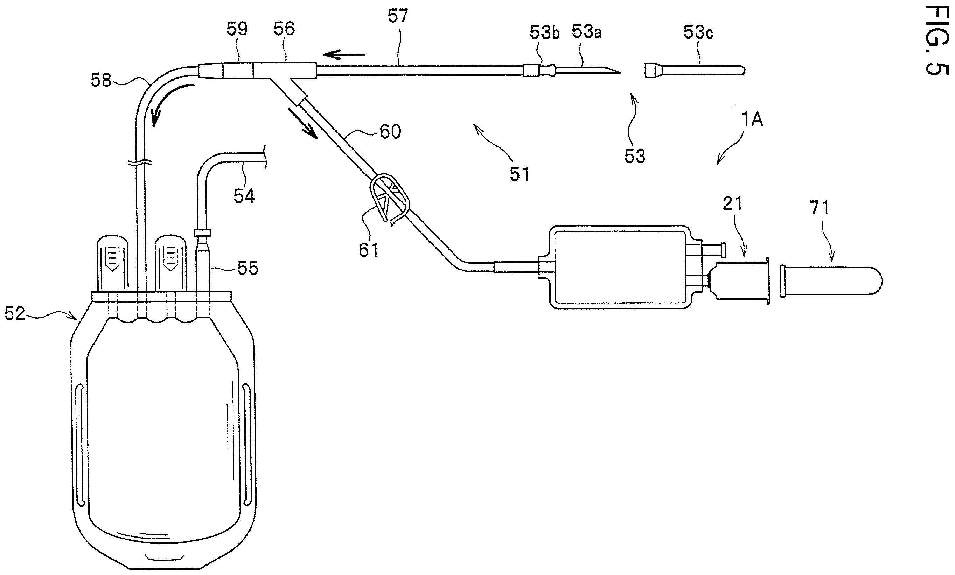

The blood inflow port 5 communicates with the internal space 3 of the main container body 2 so as to allow the initial blood flow to inflow, and is a tubular body (a tube) made of soft resin such as polyvinyl chloride. Then, one end of the blood inflow port 5 is sandwiched by the seat materials when manufacturing the main container body 2, and is joined to the sealing part 4 of the main container body 2 by fusion or adhesion so as to communicate with the internal space 3. Moreover, other end of the blood inflow port 5 is jointed to a branching tube 60 of a blood drawing instrument 51 by fusion or adhesion (see Fig. 5).

The blood outflow port 6 communicates with the internal space 3 of the main container body 2 so as to allow the initial blood flow stored in the internal space 3 to outflow to the testing instrument or the like, and is a tubular body (tube) made of soft resin such as polyvinyl chloride. Then, one end of the blood outflow port 6 is sandwiched by the seat materials when manufacturing the main container body 2, and is joined to the sealing part 4 of the main container body 2 by fusion or adhesion so as to communicate with the internal space 3. Moreover, other end of the blood outflow port 6 is jointed to the below-described blood drawing part 21 by fusion or adhesion. Incidentally, one end side of a hub 25 of a below-described needle assembly 23 (the blood drawing part 21) may be used as the blood outflow port 6, which is not illustrated, and the tubular body (the tube) may not be necessarily used.

The blood drawing part 21 is jointed to the blood outflow port 6 so as to draw the initial blood flow stored in the main container body 2 to a decompression blood drawing tube 71 (the testing instrument), and includes the needle assembly 23 and a holder 22. The needle assembly 23 is structured by: a hollow needle 24 which has a sharp needle tip on its point and is made of metal, hard resin or the like; the hub 25 which is fixed to a base end part of the hollow needle 24 and is made of polyolefin or the like; and a rubber sheath 26 that covers the hollow needle 24. Then, the hub 25 is jointed to the blood outflow port 6, whereby the hollow needle 24 communicates with the blood outflow port 6. The holder 22 is a cylindrical member made of polyolefin or the like, whose one end 22a is provided with the needle assembly 23 and whose other end 22b has an opening 22c in which the decompression blood drawing tube 71 (the testing instrument) is inserted. The one end 22a of this holder 22 is disposed on an outer periphery side of the hollow needle 24 of the needle assembly 23, and is jointed to the hub 25 concentrically with the hollow needle 24. Then, an outer diameter D_{H} of the holder 22 is set to be larger than an outer diameter D_{T} of the blood inflow port 5 (D_{H} > D_{T}).

The first discharge part 7 communicates with the internal space 3 of the main container body 2, and has a hydrophilic bacteria-impermeable first filter 12 so as to discharge air in the internal space 3, which is structured by a tubular body (tube) made of synthetic resin or the like. Incidentally, the tube is preferably made of a transparent material that facilitates confirmation of a contact of the initial blood flow with the bacteria-impermeable first filter 12. Moreover, the first discharge part 7 may be structured by a connection tube obtained by: providing a soft tube made of polyvinyl chloride or the like, which is the same material as that of the main container body 2 and facilitates fusion or adhesion, at one end side of the first discharge part 7; providing a hard tube made of polycarbonate or the like at other end of the first discharge part 7; and connecting the soft tube and the hard tube by the fusion or the adhesion, which is not illustrated.

The one end 7a of the first discharge part 7 is sandwiched by the seat materials when manufacturing the main container body 2, and is joined to the sealing part 4 of the main container body 2 by fusion or adhesion so as to communicate with the internal space 3, and then, the other end 7b of the first discharge part 7 is opened to the outside (into the atmosphere). Moreover, the hydrophilic bacteria-impermeable first filter 12 is disposed at the other end 7b of the first discharge part 7. Then, the first discharge part 7 and the blood outflow port 6 are disposed at the end part of the main container body 2 on the same side.

By disposing the first discharge part 7 and the blood outflow port 6 at the end part of the main container body 2 on the same side and jointing the blood drawing part 21 to the blood outflow port 6, the outer diameter D_{H} of the blood drawing part 21 (the holder 22) is larger than the outer diameter D_{T} of the blood inflow port 5 (D_{H} > D_{T}) while the container for testing blood 1A is in the lateral state, as shown in Figs. 2(a) to 2(c), and thus, the first discharge part 7 is disposed at a higher position (upper) than the blood inflow port 5. As a result, air, which is gathered to an upper of the internal space 3 according to the inflow of the initial blood flow, is discharged from the first discharge part 7 through the hydrophilic bacteria-impermeable first filter 12 in prior to the initial blood flow, and the inflow of the initial blood flow is automatically stopped when the initial blood flow becomes in contact with the hydrophilic bacteria-impermeable first filter 12, whereby the inflow is completed. Then, the internal space 3 is filled only with the initial blood flow, and residual air is not resulted. Also, since the initial blood flow is not in contact with the hydrophilic bacteria-impermeable first filter 12 before completing the inflow of the initial blood flow, air block of the hydrophilic bacteria-impermeable first filter 12 due to the contact of the initial blood flow is not caused, whereby stop of the inflow of the initial blood flow due to the air block can be prevented. As a result, compared with the conventional technique of stopping the inflow manually, the variation in collected amount of the initial blood flow is not caused, and the minimum collecting quantity can be secured. Also, the air entrainment is not caused according to the outflow of the initial blood flow from the blood outflow port 6 to the blood drawing part 21.

In order to obtain a higher efficiency of the above-described action in the first discharge part 7, the hydrophilic bacteria-impermeable first filter 12 is preferably a hydrophilic madreporic body, hydrophilic non-woven fabric or the like, which has a strength that withstands a venous pressure. Herein, the bacteria-impermeable first filter 12 is hydrophilic, and thus has a property of permeating gas (the air) and not permeating liquid (the initial blood flow) substantially. Thus, when the initial blood flow becomes in contact with the bacteria-impermeable first filter 12, the inflow of the initial blood flow is automatically stopped. Moreover, the bacteria-impermeable first filter 12 is hydrophilic, and thus has a property of not permeating the air after the initial blood flow becomes in contact therewith. Thus, air permeation from the bacteria-impermeable first filter 12 is blocked out, so that air tightness of the internal space 3 is maintained. As a result, while drawing the initial blood flow by connecting the decompression blood drawing tube 71 (the testing instrument) to the below-described blood drawing part 21, a decompression state of the decompression blood drawing tube can be maintained, and the initial blood flow can be sucked securely into the decompression blood drawing tube. As the bacteria-impermeable first filter 12, a madreporic body, non-woven fabric or the like which is made of a hydrophilic material such as acrylic resin is exemplified, and alternatively, a madreporic body, non-woven fabric or the like, whose surface is covered with a hydrophilic material such as acrylic resin by a plasma treatment or the like, may be used. Incidentally, a pore diameter of the bacteria-impermeable first filter 12 preferably ranges from 0.01 µm to 100 µm. When the pore diameter is smaller than 0.01 µm, the permeability is lacked easily, and when the pore diameter is larger than 100 µm, bacterial penetration is hard to be suppressed.

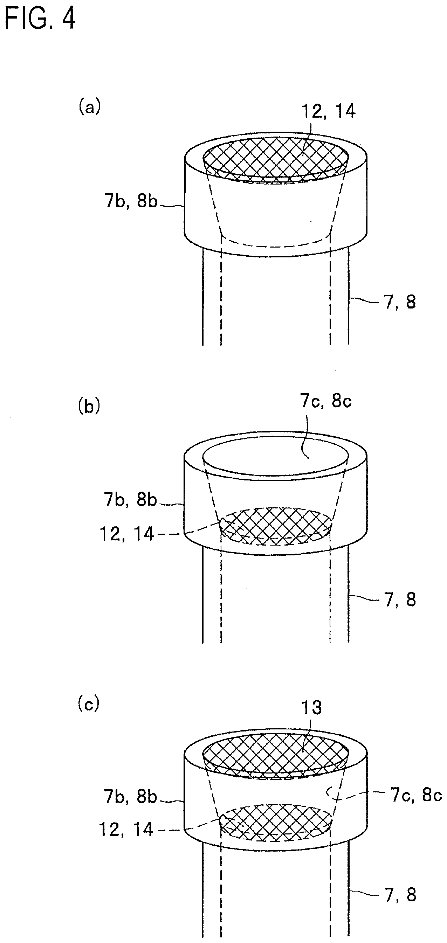

As shown in Fig. 4(a), the hydrophilic bacteria-impermeable first filter 12 is disposed at a tip part of the other end 7b of the tube 7 which is opened to the outside (into the atmosphere), where it is preferable to provide a concave part 7c at the tip part of the other end 7b, and dispose the hydrophilic bacteria-impermeable first filter 12 at a bottom part of the concave part 7c, as illustrated in Fig. 4 (b) . Alternatively, as shown in Fig. 4 (c), it is possible to provide the concave part 7c at the tip part of the other end 7b, and dispose the hydrophilic bacteria-impermeable first filter 12 at the bottom part of the concave part 7c, and further, cover the tip part (an upper surface part of the concave part) of the other end 7b with a covering member 13 made of non-woven fabric or the like. By structuring the bacteria-impermeable first filter 12 of the first discharge part 7 as illustrated in Figs. 4 (b) and 4 (c), an operator can be prevented from touching the bacteria-impermeable first filter 12 by mistake. As a result, the bacteria-impermeable first filter 12 can be also prevented from getting wet by the touch of the operator, and thus can be prevented from deterioration of the function of discharging the air.

The first discharge part 7 preferably has a tube length L_{T} of 20 mm or more. By lengthening the tube length of L_{T} of the first discharge part 7, the contact of the initial blood flow with the bacteria-impermeable first filter 12 can be prevented more. Incidentally, an upper limitation of the tube length of L_{T} is not particularly determined, but when the tube length of L_{T} of the first discharge part 7 is too long, the container for testing blood 1A becomes less usable, and thus 70 mm or less is preferable. Further, as illustrated in Fig. 2(b), the first discharge part 7 and the blood drawing part 21 are structured by different members. However, as shown in Fig. 2 (c), the first discharge part 7 and the blood drawing part 21 may be connected integrally via a connecting part 9 so as to be structured by the same member. Alternatively, the first discharge part 7 and the blood outflow port 6 may be connected integrally via the connecting part 9, which is not illustrated.

Next, another embodiment of the container for testing blood will be described with reference to the accompanying drawings.

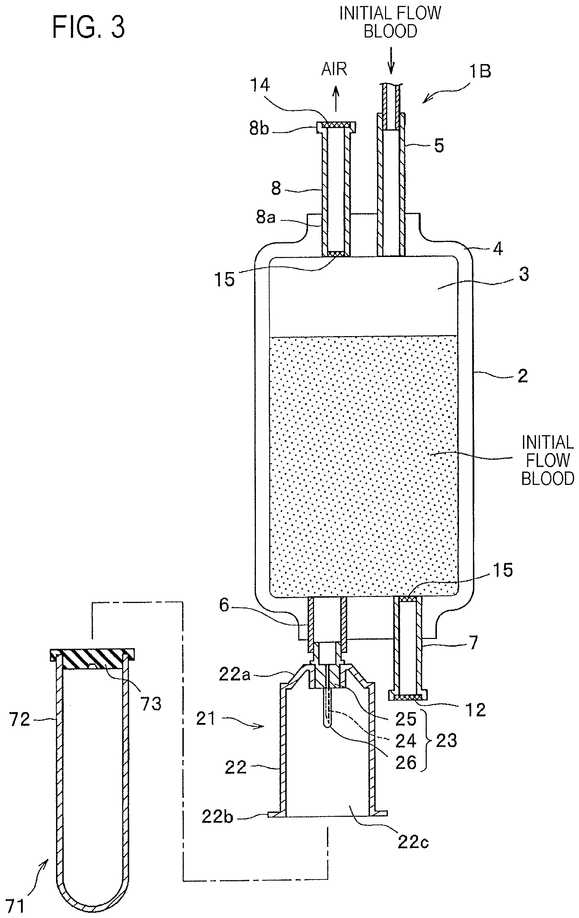

As illustrated in Fig. 3, a container for testing blood 1B includes a second discharge part 8 in addition to the above-described constituents.

The second discharge part 8 communicates with the internal space 3 of the main container body 2 and has a hydrophilic bacteria-impermeable second filter 14 so as to discharge the air in the internal space 3, which is structured by a tubular body (a tube) made of synthetic resin or the like. Incidentally, the tube is preferably made of a transparent material that facilitates confirmation of a contact of the initial blood flow with the bacteria-impermeable second filter 14. Moreover, a tube length of the second discharge part 8 is similar to the tube length L_{T} of the first discharge part 7.

One end 8a of the second discharge part 8 is sandwiched by the seat materials when manufacturing the main container body 2, and is joined to the sealing part 4 of the main container body 2 by fusion or adhesion so as to communicate with the internal space 3, and then, other end 8b of the second discharge part 8 is opened to the outside (into the atmosphere). Moreover, the second discharge part 8 may be structured by a connection tube obtained by connecting a soft tube and a hard tube by fusion or adhesion similarly to the first discharge part 7, which is not illustrated. Further, as shown in Figs. 4(a) to 4(c), the hydrophilic bacteria-impermeable second filter 14 is disposed at a tip part of the other end 8b of the second discharge part 8, or at a concave part 8c that is provided at the tip part of the other end 8b. Incidentally, a constituent material and the like of the bacteria-impermeable second filter 14 are similar to those of the bacteria-impermeable first filter 12.

The second discharge part 8 is disposed at an end part of the main container body 2 on an opposite side of the first discharge part 7, and when using the container for testing blood 1B so that the blood outflow port 6 is directed vertically, that is, when the container for testing blood 1B is hung down from a stand or the like so as to be in the drooping state, the second discharge part 8 is disposed at the upper end side of the main container body 2. Then, since the second discharge part 8 has the hydrophilic bacteria-impermeable second filter 14, the air, which is gathered to the upper of the internal space 3 according to the inflow of the initial blood flow, is discharged from the second discharge part 8, and the inflow of the initial blood flow is automatically stopped when the initial blood flow becomes in contact with the bacteria-impermeable second filter 14. Then, the internal space 3 is filled only with the initial blood flow, and the residual air is not resulted. As a result, compared with the conventional technique of stopping the inflow manually, the variation in collected amount of the initial blood flow is not caused, and the minimum collecting quantity can be secured. Moreover, the air entrainment is not caused according to the outflow of the initial blood flow from the blood outflow port 6 to the blood drawing part 21.

As shown in Figs. 1 and 3, in the containers for testing blood 1A and 1B, the first discharge part 7 and the second discharge part 8, among the above-described constituents, preferably include defoaming filters 15 on the internal space 3 sides of the bacteria-impermeable first filter 12 and the bacteria-impermeable second filter 14, respectively. Moreover, the defoaming filters 15 are preferably disposed near a boundary between the first discharge part 7 and the internal space 3 and a boundary between the second discharge part 8 and the internal space 3, respectively.

As the defoaming filter 15, a foam body such as foamed polyurethane, foamed polyethylene, foamed polypropylene and foamed polystyrene, mesh, woven fabric, non-woven fabric, or various porous bodies such as porous ceramics and a sintered body of resin or the like can be used preferably. Moreover, a pore diameter of the defoaming filter 15 preferably ranges from 20 µm to 10 mm, and particularly preferably ranges from about 50 µm to about 5 mm.

Further, in the defoaming filter 15, a defoaming agent that has a function of breaking an air bubble, when the air bubble becomes in touch, is preferably supported by the porous body or the like. As this defoaming agent, silicone (a compound type in which silica is blended, an oil type or the like) is preferably used. Moreover, a method for allowing the defoaming agent to be supported by the porous body or the like may be: impregnating the porous body with liquid containing the defoaming agent; applying or spraying the liquid containing the defoaming agent to the porous body or the like and subsequently drying; or the like.

Next, an embodiment of the blood drawing instrument according to the present invention will be described.

As shown in Fig. 5, the blood drawing instrument 51 (a blood bag system) includes: a blood drawing needle 53; a blood collecting bag 52 (the storage part, a blood bag) that stores the blood collected through the blood drawing needle 53; a blood drawing line that connects the blood drawing needle 53 with the blood collecting bag 52; a branching line that branches from a midway of the blood drawing line; and the container for testing blood 1A that communicates with the blood drawing line via the branching line and draws (stores) the initial blood flow. Hereinafter, explanations of the respective constituents will be provided. Incidentally, since the container for testing blood 1A is as described above, the explanation thereof will be omitted. Moreover, the blood drawing instrument 51 may be provided with the container for testing blood 1B instead of the container for testing blood 1A, which is not illustrated.

The blood drawing needle 53 is structured by: a hollow needle 53a which has a sharp needle tip on its point and is made of metal, hard resin or the like; a hub 53b which is fixed to a base end part of the hollow needle 53a and is made of polyolefin or the like; and a protector 53c which covers the hollow needle 53a and is made of hard resin or the like.

The blood collecting bag 52 is a bag-shaped container obtained by superimposing resin seats that are made of polyvinyl chloride or the like and fusing or adhering peripheries of the superimposed resin seats, and inside the blood collecting bag 52, blood after removing (collecting) the initial blood flow from the blood that is drawn through the blood drawing needle 53 is stored, as stated in a below-described blood processing method. Moreover, inside the blood collecting bag 52, anticoagulant is preferably stored in advance.

To a top part of the blood collecting bag 52, one end of a tube 54 is connected via a sealing member 55. Then, to other end of the tube 54, the blood bag such as a leukocyte removing filter, a collecting bag (a red cell collecting bag), a blood plasma collecting bag and a red cell preservative solution containing bag is connected, which is not illustrated. Incidentally, as the leukocyte removing filter, a conventionally well-known filter is used, and each of the collecting bag, the blood plasma collecting bag and the red cell preservative solution containing bag is a bag-shaped container that is made of polyvinyl chloride similarly to the blood collecting bag 52.

Moreover, to the top part of the blood collecting bag 52, one end of a tube 58 is connected. Then, other end of the tube 58 is connected to a branching connector 56, and the branching connector 56 is connected to the blood drawing needle 53 (the hub 53b) via a tube 57. Thus, the tubes 57 and 58 and the branching connector 56 construct the blood drawing line, and the blood collecting bag 52 and the blood drawing needle 53 are connected with each other via the blood drawing line. Further, other end of the branching tube 60, whose one end is connected to the container for testing blood 1A, is connected to the branching connector 56 so as to construct the branching line that branches from the midway of the blood drawing line, and the container for testing blood 1A communicates with the blood drawing line via the branching line. Moreover, between the branching connector 56 and the tube 58, a sealing member 59 is provided so that the initial blood flow may not inflow to the tube 58 on the blood collecting bag 52 side, when drawing (storing) the initial blood flows into the container for testing blood 1A through the branching tube 60. Further, the branching tube 60 is provided with a clamp 61 for closing a blood drawing channel of the initial blood flow.

Incidentally, each of the sealing members 55 and 59 is structured by a short tube and a cylindrical body having a solid tip part stored therein for closing the channel (not illustrated), and each of the sealing members 55 and 59 is a member for opening the channel in the short tube by breaking the cylindrical solid tip part. Further, the short tube is made of a soft material such as polyvinyl chloride, and the cylindrical body is made of a hard material such as polycarbonate. Incidentally, the branching connector 56, the tubes 54, 57 and 58 and the branching tube 60 are made of polyvinyl chloride or the like.

Next, the blood processing method using the above-described container for testing blood and the blood drawing instrument that have been described above will be explained. Herein, the structure of the container for testing blood will be referred to Figs. 1, 2(a) to 2(c) and 3 as appropriate.

Firstly, as shown in Fig. 5, in the blood drawing instrument 51, since the clamp 61 is opened state, a blood drawing channel of the branching tube 60 is opened state. Then, a blood drawing channel of the tube 58 is closed by the sealing member 59. Moreover, the whole of the container for testing blood 1A is placed horizontally (in the lateral state) so that the surface of the container is in contact with the work table or the like (see Figs. 2 (a) to 2(c)). Incidentally, in the case of the container for testing blood 1B, the container for testing blood 1B is hung down from the stand or the like so as to be in the drooping state, which is not illustrated, so that the second discharge part 8 is positioned at the upper end of the main container body 2 (see Fig. 3).

In this state, a vain of a donor is punctured with the blood drawing needle 53. Thereby, the initial blood flow passes through the blood drawing needle 53, the tube 57 and the branching connector 56, and inflows into the branching tube 60, and then, is collected (stored) into the container for testing blood 1A. In this case, since the blood drawing channel of the tube 58 is blocked out by the sealing member 59 as described above, the initial blood flow flows from the tube 57 through the branching connector 56 into the branching tube 60 securely. Then, air in the tube 57, the branching connector 56 and the branching tube 60 is discharged into the container for testing blood 1A (the main container body 2) in prior to the inflow of the initial blood flow. Incidentally, the case of the container for testing blood 1B is similar to the above-described case of the container for testing blood 1A.

In the container for testing blood 1A, the initial blood flow inflows from the blood inflow port 5 into the internal space 3 of the main container body 2, as shown in Figs. 2 (a) to 2(c) . Then, the air in the internal space 3 is gathered to the upper according to the inflow of the initial blood flow, and is discharged through the hydrophilic bacteria-impermeable first filter 12 of the first discharge part 7 to the outside (into the atmosphere). Then, after visually confirming that the initial blood flow is in contact with the hydrophilic bacteria-impermeable first filter 12 and the inflow of the initial blood flow is automatically stopped, the clamp 61 is closed so as to close the blood drawing channel of the branching tube 60, thereby stopping the inflow of the initial blood flow from the blood inflow port 5. Incidentally, in the container for testing blood 1B, as shown in Fig. 3, the air in the internal space 3 is discharged through the hydrophilic bacteria-impermeable second filter 14 of the second discharge part 8 to the outside (into the atmosphere), and the initial blood flow becomes in contact with the hydrophilic bacteria-impermeable second filter 14, whereby the inflow of the initial blood flow is stopped automatically.

Subsequently, blood collection (actual blood collection) into the blood collecting bag 52 is started.

In this case, by breaking a solid tip part (not illustrated) of the sealing member 59, the channel inside the sealing member 59 is opened. By this operation, the blood drawing channel of the tube 58 becomes opened state, so that the tube 57 and the tube 58 communicate with each other. Thereby, the drawn blood passes through the tube 57, the branching connector 56, the sealing member 59 and the tube 58, and then inflows into the blood collecting bag 52.

Moreover, judging from a condition of the donor and the blood withdrawal, if possible, the initial blood flow collected (stored) in the container for testing blood 1A is sampled into the testing instrument such as the decompression blood drawing tube 71, in parallel with this blood withdrawal into the blood collecting bag 52.

For sampling, as shown in Fig. 1, the decompression blood drawing tube 71 is inserted into the blood drawing part 21 (the holder 22) until reaching an innermost part of the holder 22, and a rubber stopper 73 that is engaged in a blood drawing tube main body 72 of the decompression blood drawing tube 71 is punctured with the hollow needle 24 so as to be penetrated thereby. Thereby, the initial blood flow stored in the container for testing blood 1A is sucked into the blood drawing tube main body 72 so as to be sampled. Thereafter, the decompression blood drawing tube 71 is pulled out of the blood drawing part 21 (the holder 22). Moreover, when sampling the initial blood flow into a plurality of the decompression blood drawing tube 71, this operation is to be repeated. Incidentally, the case of the container for testing blood 1B is similar to the above-described case of the container for testing blood 1A.

Further, as shown in Figs. 4(a) to 4(c), while drawing the blood into the blood collecting bag 52, after collecting a preset amount of the blood into the blood collecting bag 52, the blood drawing needle 53 is pulled out of the vein of the donor, and the tube 58 and the branching tube 60 are closed and sealed by a tube sealer or the like as necessary. Thereafter, the container for testing blood 1A and the blood drawing needle 53 are separated. Thereby, the blood collecting bag 52 storing the blood from which the initial blood flow is removed can be obtained. Incidentally, the case of the container for testing blood 1B is similar to the above-described case of the container for testing blood 1A.

Moreover, the blood stored in the blood collecting bag 52 is allowed to pass through the leukocyte removing filter, which is not illustrated, so as to separate a leukocyte and a platelet, and the remaining blood component is collected into the collecting bag, and thereafter, the blood collecting bag 52 and the leukocyte removing filter are separated. Subsequently, the blood in the collecting bag is centrifuged so as to be separated into a red cell layer and a blood plasma layer, and after transferring the blood plasma into a blood plasma bag, the red cell preservative solution in the red cell preservative solution containing bag is added to the concentrated red cells that remain in the collecting bag, and is mixed therewith.

The embodiments of the container for testing blood and the blood drawing instrument of the present invention have been described above, but the present invention is not limited to the above-described embodiments, and, for example, a blood separator (for example, a centrifugal separator and a membrane separator) may be provided, instead of the blood bag, as the storage part in the blood drawing instrument of the present invention. That is, the blood drawing instrument is not limited to the blood bag system, but may be an apheresis collection set.

### Reference Signs List

1A, 1B container for testing blood
2 main container body
3 internal space
5 blood inflow port
6 blood outflow port
7 first discharge part
8 second discharge part
12 bacteria-impermeable first filter
14 bacteria-impermeable second filter
21 blood drawing part
51 blood drawing instrument
52 blood collecting bag (storage part)
53 blood drawing needle

## Claims

1. A container (1A; 1B) for testing blood comprising:
a main container body (2) having an internal space (3) that stores an initial blood flow to be used for a test;
a blood inflow port (5) that communicates with the internal space (3) and allows the initial blood flow to inflow;
a blood outflow port (6) that communicates with the internal space (3) and allows the stored initial blood flow to outflow;
a blood drawing part (21) that is connected to the blood outflow port (6) and allows the initial blood flow to be drawn; and
a first discharge part (7) that communicates with the internal space (3), has a hydrophilic bacteria-impermeable first filter (12), and discharges air in the internal space (3), whereby
the first discharge part (7) and the blood outflow port (6) are disposed at an end part of the main container body (2) on the same side, and
the blood drawing part (21) connected to the blood outflow port (6) includes:
a needle assembly (23) having a hollow needle (24) that communicates with the blood outflow port (6); and
a cylindrical holder (22) whose one end is provided with the needle assembly (23) and whose other end has an opening in which a testing instrument storing the initial blood flow is inserted,
**characterized in that**
while the container (1A; 1B) for testing blood is in an horizontally placed state in which the whole of the container (1A; 1B) for testing blood is placed horizontally so that a surface of the container (1A; 1B) for testing blood is in contact with a work table or the like, the blood outflow port (6) is disposed at a higher position than the blood inflow port (5).

2. The container (1A; 1B) for testing blood according to claim 1, wherein the first discharge part (7) further includes a defoaming filter (15) on the internal space side of the bacteria-impermeable first filter (12).

3. The container (1B) for testing blood according to claim 1, further comprising a second discharge part (8) which is disposed at an end part of the main container body (2) on an opposite side of the first discharge part (7), which is disposed at an upper end side of the main container body (2) when being used so that the blood outflow port (6) is directed vertically, and which communicates with the internal space (3), has a hydrophilic bacteria-impermeable second filter (14), and discharges the air in the internal space (3).

4. The container (1B) for testing blood according to claim 3, wherein the second discharge part (8) further comprises a defoaming filter (15) on the internal space side of the bacteria-impermeable second filter (14).

5. A blood drawing instrument (51) comprising:
a blood drawing needle (53); a storage part (52) that stores blood drawn through the blood drawing needle (53);
a blood drawing line (57, 56, 59, 58) that connects the blood drawing needle (53) with the storage part (52); a branching line (60) that branches from a midway of the blood drawing line (57, 56, 59, 58); and
the container (1A; 1B) for testing blood according to any one of claims 1 to 4, which communicates with the blood drawing line (57, 56, 59, 58) via the branching line (60).

6. The blood drawing instrument according to claim 5, wherein the storage part is a blood bag (52) or a blood separator.

## Patentansprüche

1. Behälter (1A; 1B) zum Untersuchen von Blut mit:
einem Hauptbehälterkörper (2), der einen Innenraum (3) aufweist, der einen Anfangsblutstrom speichert, der für einen Versuch zu verwenden ist;
einem Bluteinströmanschluss (5), der mit dem Innenraum (3) in Verbindung ist und es dem Anfangsblutstrom gestattet, einzuströmen;
einem Blutausströmanschluss (6), der mit dem Innenraum (3) in Verbindung ist und dem gespeicherten Anfangsblutstrom gestattet, auszuströmen;
einem Blutziehteil (21), das mit dem Blutausströmanschluss (6) in Verbindung ist und dem Anfangsblutstrom gestattet, gezogen zu werden; und
einem ersten Abgabeteil (7) das mit dem Innenraum (3) in Verbindung ist, einen hydrophilen bakterienundurchlässigen ersten Filter (12) aufweist und Luft in den Innenraum (3) abgibt,
wobei
das erste Abgabeteil (7) und der Blutausströmanschluss (6) an einem Endteil des Hauptbehälterkörpers (2) an der gleichen Seite vorgesehen sind, und
das Blutziehteil (21), das mit dem Blutausströmanschluss (6) verbunden ist, hat:
eine Nadelbaugruppe (23), die eine hohle Nadel (24) aufweist, die mit dem Blutausströmanschluss (6) in Verbindung ist; und
einen zylindrischen Halter (22), dessen eines Ende mit der Nadelbaugruppe (23) bereitgestellt ist, und dessen anderes Ende eine Öffnung aufweist, in der ein Untersuchungsinstrument einzufügen ist, das den Anfangsblutstrom speichert,
**dadurch gekennzeichnet, dass**
während der Behälter (1A; 1B) zum Untersuchen des Bluts sich in einem horizontal platzierten Zustand befindet, in dem das Gesamte des Behälters (1A; 1B) zum Untersuchen des Bluts horizontal platziert ist, so dass eine Oberfläche des Behälters (1A; 1B) zum Untersuchen des Bluts mit einem Arbeitstisch oder Ähnlichem in Berührung ist, der Blutausströmanschluss (6) an einer höheren Position als der Bluteinströmanschluss (5) vorgesehen ist.

2. Behälter (1A; 1B) zum Untersuchen von Blut nach Anspruch 1, wobei das erste Abgabeteil (7) außerdem einen Entschäumungsfilter (15) an der Innenraumseite des bakterienundurchlässigen ersten Filters (12) hat.

3. Behälter (1B) zum Untersuchen von Blut nach Anspruch 1, außerdem mit einem zweiten Abgabeteil (8), das an einem Endteil des Hauptbehälterkörpers (2) an einer gegenüberliegenden Seite des ersten Abgabeteils (7) vorgesehen ist, das an einer oberen Endseite des Hauptbehälterkörpers (2) vorgesehen ist, wenn es verwendet wird, so dass der Blutausströmanschluss (6) vertikal gerichtet ist, und das mit dem Innenraum (3) in Verbindung ist, einen hydrophilen bakterienundurchlässigen zweiten Filter (14) aufweist, und die Luft in dem Innenraum (3) abgibt.

4. Behälter (1B) zum Untersuchen von Blut nach Anspruch 3, wobei das zweite Abgabeteil (8) außerdem einen Entschäumungsfilter (15) auf der Innenraumseite des bakterienundurchlässigen zweiten Filters (14) umfasst.

5. Blutziehinstrument (51) mit:
einer Blutziehnadel (53); einem Speicherteil (52), das durch die Blutziehnadel (53) gezogenes Blut speichert;
einer Blutziehleitung (57, 56, 59, 58), die die Blutziehnadel (53) mit dem Speicherteil (52) verbindet; einer Zweigleitung (60), die von einer Mitte der Blutziehleitung (57, 56, 59, 58) abzweigt; und
dem Behälter (1A; 1B) zum Untersuchen von Blut gemäß einem der Ansprüche 1 bis 4, der mit der Blutziehleitung (57, 56, 59, 58) über die Zweigleitung (60) in Verbindung ist.

6. Blutziehinstrument nach Anspruch 5, wobei das Speicherteil ein Blutbeutel (52) oder ein Blutseparator ist.

## Revendications

1. Récipient (1A ; 1B) pour analyser du sang comprenant :
un corps de récipient principal (2) ayant un espace interne (3) qui stocke un écoulement de sang initial destiné à être utilisé pour une analyse ;
un orifice d'entrée de sang (5) qui communique avec l'espace interne (3) et permet à l'écoulement de sang initial d'entrer en s'écoulant ;
un orifice de sortie de sang (6) qui communique avec l'espace interne (3) et permet à l'écoulement de sang initial stocké de sortir en s'écoulant ;
une partie d'aspiration de sang (21) qui est raccordée à l'orifice de sortie de sang (6) et permet à l'écoulement de sang initial d'être aspiré ; et
une première partie de décharge (7) qui communique avec l'espace interne (3), a un premier filtre hydrophile imperméable aux bactéries (12) et décharge l'air dans l'espace interne (3), moyennant qui :
la première partie de décharge (7) et l'orifice de sortie de sang (6) sont disposés au niveau d'une partie d'extrémité du corps de récipient principal (2) du même côté, et
une partie d'aspiration de sang (21) raccordée l'orifice de sortie de sang (6) comprend :
un ensemble d'aiguille (23) ayant une aiguille creuse (24) qui communique avec l'orifice de sortie de sang (6) ; et
un support cylindrique (22) dont une extrémité est prévue avec l'ensemble d'aiguille (23) et dont l'autre extrémité a une ouverture dans laquelle un instrument d'analyse stockant l'écoulement de sang initial est inséré,
**caractérisé en ce que** :
alors que le récipient (1A ; 1B) pour analyser du sang est dans un état placé horizontalement dans lequel la totalité du récipient (1A ; 1B) pour analyser du sang est placé horizontale de sorte qu'une surface du récipient (1A ; 1B) pour analyser du sang est en contact avec un plan de travail ou similaire, l'orifice de sortie de sang (6) est disposé dans une position plus haute que l'orifice d'entrée de sang (5).

2. Récipient (1A ; 1B) pour analyser du sang selon la revendication 1, dans lequel la première partie de décharge (7) comprend en outre un filtre antimousse (15) du côté de l'espace interne du premier filtre imperméable aux bactéries (12).

3. Récipient (1B) pour analyser du sang selon la revendication 1, comprenant en outre une seconde partie de décharge (8) qui est disposée au niveau d'une partie d'extrémité du corps de récipient principal (2) sur un côté opposé de la première partie de décharge (7) qui est disposée au niveau d'un côté d'extrémité supérieur du corps de récipient principal (2) lorsqu'elle est utilisée de sorte que l'orifice de sortie de sang (6) est dirigé verticalement et qui communique avec l'espace interne (3), a un second filtre hydrophile imperméable aux bactéries (14) et décharge l'air dans l'espace interne (3).

4. Récipient (1B) pour analyser du sang selon la revendication 3, dans lequel la seconde partie de décharge (8) comprend en outre un filtre antimousse (15) du côté de l'espace interne du second filtre imperméable aux bactéries (14).

5. Instrument d'aspiration de sang (51) comprenant :
une aiguille d'aspiration de sang (53) ; une partie de stockage (52) qui stocke le sang aspiré par l'aiguille d'aspiration de sang (53) ;
une ligne d'aspiration de sang (57, 56, 59, 58) qui raccorde l'aiguille d'aspiration de sang (53) avec la partie de stockage (52) ; une ligne de dérivation (60) qui part à mi-chemin de la ligne d'aspiration de sang (57, 56, 59, 58) ; et
le récipient (1A ; 1B) pour analyser du sang selon l'une quelconque des revendications 1 à 4, qui communique avec la ligne d'aspiration de sang (57, 56, 59, 58) via la ligne de dérivation (60).

6. Instrument d'aspiration de sang selon la revendication 5, dans lequel la partie de stockage est un sachet de sang (52) ou un séparateur de sang.
